(19) Europäisches Patentamt
European Patent Office
Office européen des brevets

(11) Veröffentlichungsnummer: **0 212 293**
**A2**

# (12) EUROPÄISCHE PATENTANMELDUNG

(21) Anmeldenummer: **86110026.1**

(22) Anmeldetag: **22.07.86**

(51) Int. Cl.⁴: **C07D 311/92**, A61K 31/35, C12P 17/06

(30) Priorität: **31.07.85 DE 3527336**

(43) Veröffentlichungstag der Anmeldung:
**04.03.87 Patentblatt 87/10**

(84) Benannte Vertragsstaaten:
**AT BE CH DE FR GB IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Aretz, Werner, Dr.**
**Am Krummorgen 2**
**D-6233 Kelkheim (Taunus)(DE)**
Erfinder: **Böttger, Dirk, Dr.**
**Bahnstrasse 4**
**D-6237 Liederbach(DE)**
Erfinder: **Sauber, Klaus, Dr.**
**Friedrich-Ebert-Strasse 15**
**D-6231 Schwalbach am Taunus(DE)**

## (54) Verfahren zur mikrobiellen Hydroxylierung von Forskolin und seinen Derivaten durch Neurospora crassa.

(57) Die vorliegende Erfindung betrifft Forskolin-Analoge und Derivate und ein Verfahren zu deren Herstellung mittels mikrobieller Umwandlungen durch Mikroorganismen des Stammes Neurospora crassa.

Die erfindungsgemäß hergestellten Verbindungen haben vielfältige pharmakologische Wirkungen und können daher als Arzneimittel verwendet werden.

EP 0 212 293 A2

# Verfahren zur mikrobiellen Hydroxylierung von Forskolin und seinen Derivaten durch Neurospora crassa

Die vorliegende Erfindung betrifft Forskolin-Analoge und Derivate, ein Verfahren zu deren Herstellung mittels mikrobieller Umwandlungen durch Neurospora crassa und die Verwendung der Substanzen als pharmazeutische Mittel, insbesindere als Medikamente zur Behandlung von Glaukom, Herzinsuffizienz, Hypertonie, Entzündung, Allergien, Bronchialerkrankungen und Erkrankungen des Immunsystems.

Forskolin und seine natürlich vorkommenden Analoge stellen eine Gruppe von Labdan-Terpenoiden dar, die aus der Pflanze Coleus forskolii - (Briqu.) isoliert werden. Die Strukturformeln von Forskolin und seinen natürlich vorkommenden, isolierten Analogen werden in der Formel I dargestellt.

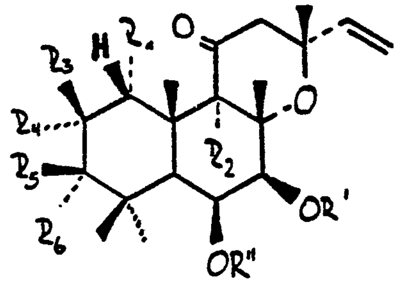

Für Forskolin ist $R_1$, $R_2$ = OH, R' = Ac, sowie $R_3$ bis $R_7$ und R" = H

Bis heute sind folgende natürlich vorkommenden Forskolin-Analoge nachgewiesen worden:

1,9-Didesoxyforskolin: R' = Ac, sowie $R_1$ bis $R_7$ und R" = H

9, Desoxyforskolin: $R_1$ = OH, R' = Ac, sowie $R_2$ bis $R_7$ und R" = H

1,9-Didesoxy-7-deacetyl-forskolin: R', R" und $R_1$ bis $R_7$ = H

1-Desoxyforskolin: $R_2$ = OH, R' = Ac, sowie R" und $R_1$, $R_3$ bis $R_7$ = H

7-Deacetylforskolin: $R_1$, $R_2$ = OH, R', R", sowie $R_3$ bis $R_7$ = H

6-Acetyl-7-deacetyl-forskolin: $R_1$ = OH, R" = Ac, sowie R' und $R_2$ bis $R_7$ = H

(Tetrahedron Lett. 19, 1669 (1977); J. Med. Chem. 26, 486 (1983).

Forskolin ist eines der wichtigen Diterpene der Pflanze Coleus forskolii. Diese Substanz und seine Derivate können verwendet werden als wertvolle Arzneimittel bei der Behandlung verschiedener Erkrankungen wie Glaukom, Hypertonie, Herzinsuffizienz, Entzündung, Allergien, Bronchokonstriktion und ganz allgemein von Erkrankungen, die durch Störungen der cAMP-Produktion und -Metabolismus hervorgerufen werden (Indisches Patent Nr. 143 875 und entsprechende DE-OS 25 57 784, Indisches Patent Nr. 145 926, Indisches Patent Nr. 147 630 und entsprechende DE-OS 26 40 275; Med. Res. Revs. 3, 201-19 (1983)).

Ein Verfahren zur Isolierung des Hauptanteils von Forskolin aus Coleus forskohlii ist bereits beschreiben (s. Indische Patente Nr. 143 875 und 145 926, DE-OS 25 57 784). Bei diesem Verfahren werden außerdem Forskolin-Analoge gewonnen. Diese Analoge können verwendet werden als Zwischenprodukte zur Herstellung von Forskolin. Es sind bereits Methoden beschrieben worden, durch die die Analoge mit gleicher Oxidationsstufe wie Forskolin, z.B. 7-DeacetylForskolin und 6-Acetyl-7-deacetylforskolin in Forskolin umgewandelt werden können (Indisches Patent 147 007, J. Chem. Perk. Trans. 1, 767 (1982)).

Es ist bereits ein Verfahren der mikrobiellen Umwandlung beschrieben worden, durch das die weniger hydroxylierten Forskolin-Analoge in Forskolin, 7-Deacetyl-forskolin oder 6-Acetyl-7-deacetylforskolin, oder in solche Derivate umgewandelt werden, die sich durch bekannte chemische Methoden leicht in Forskolin umwandeln lassen (Deutsche Patentanmeldung P 35 02 685.5).

Gegenstand der vorliegenden Erfindung ist nun ein mikrobielles Verfahren zur Hydroxylierung von Labdan-Terpenoiden der Formel I

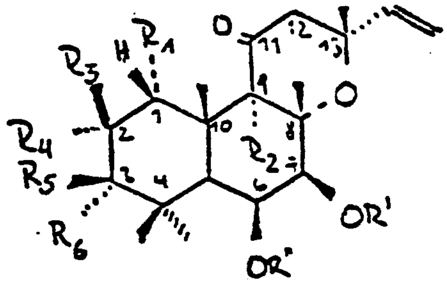

worin

$R_1$ und $R_2$ Wasserstoff oder Hydroxy, $R_3$ bis $R_6$ Wasserstoff, R' die Acetylgruppe oder Wasserstoff und R" Wasserstoff bedeuten, durch Neurospora-Stämme, insbesondere durch die Stämme Neurospora crassa ATCC 9279 (hinterl. 5.12.1944), ATCC 9344 (hinterl. 16.6.1947), ATCC 10336 (hinterl. 4.3.1948) und ATCC 10780 (hinterl. 31.5.1950). Das Verfahren ist dadurch gekennzeichnet, daß man eine Verbindung der Formel I, worin $R_1$-$R_6$, R' und R" die obengenannten Bedeutungen haben, in einem üblichen Nährmedium definierter Zusammensetzung, das einen der genannten Mikroorganismen-Stämme enthält, fermentiert. Die Fermentation erfolgt 3 -5 Tage lang bei einer Temperatur von 25 -30°C, bevorzugt bei 28°C unter Schütteln.

Das Nährmedium enthält Kohlenstoff-und Stickstoffquellen, gegebenenfalls auch anorganische Nährsalze sowie Spurenelemente.

Als Kohlenstoffquellen eignen sich z.B. Glucose, Stärke, Dextrin und Glycerin. Geeignete Stickstoffquellen sind z.B. Sojamehl, Hefeextrakt, Fleischextrakt, Malzextrakt, Maisquellwasser, Pepton und Casein. Als anorganische Nährsalze kommen z.B. Natriumchlorid, Magnesiumsulfat oder Calciumcarbonat, als Spurenelemente z.B. Eisen, Magnesium, Kupfer, Zink und Kobalt in Form geeigneter Salze in Frage.

Nach Abschluß der Umsetzung wird das Pilzmycel abfiltriert und das resultierende Kultur-Filtrat mit organischen Lösungsmitteln wie Dichlormethan, Chloroform oder Äthylacetat extrahiert. Der Extrakt wird zweckmäßig über Trocknungsmitten wie $Na_2SO_4$ getrocknet und im Vakuum konzentriert. Der Rückstand wird dann durch Trennungsverfahren wie beispielsweise Chromatographie und durch Kristallisation gereinigt.

Die Hydroxylierung erfolgt bevorzugt in 1-, 2- und/oder 3-Stellung.

Allgemeine Verfahrensbeschreibung:

a) Stammhaltung und Anzucht

Sporensuspensionen der obengenannten Stämme werden auf Schrägröhrchen mit folgendem nährmedium beimpft und 14 Tage bei 30°C bebrütet:

Stammhaltungsnährmedium:

Glucose 10 g/l

Caseinpepton 4 g/l

Fleischextrakt 4 g/l

NaCl 2,5 g/l

Hefeextrakt 0,5 g/l

Leberextrakt 0,5 g/l

Agar 20 g/l

pH 7,2 -7,5

Zur Anzucht in Flüssigkulturen werden von den Stämmen Sporensuspensionen durch Abschwemmen der Schrägröhrchen mit steriler NaCl 0.8 % gewonnen und diese auf einen Titer von 1 x $10^5$ Sporen/ml verdünnt. 0.5 ml dieser Suspension dienen als Inokulum von 50 ml Hauptkultur (in 100 ml Erlenmeyerkolben) folgender Zusammensetzung:

Hauptkulturnährlösung:

Glucose 40 g/l

Pepton 10 g/l

Hefeextrakt 2 g/l

Cornsteep fl 2 ml

pH 5,5

Nach 30 -40 Stunden bei 29°C und einer Schüttelfrequenz von 190 Upm erzielt man ein dichtes, mycelartiges Wachstum.

b) Biotransformation

Zur Biotransformation werden die 50 ml Hauptkultur in einen sterilen 300 ml Erlenmeyerkolben überführt und 16 mg Substrat (in Methanol gelöst) zugesetzt. Anschliessend wird die Suspension bei 28°C und 240 Upm 3 -5 Tage geschüttelt. Der Fortgang der Biotransformation wird mittels Dünnschichtchromatographie (HPTCL-Platten, Methylenchlorid/Ethylacetat = 3 / l: Anisaldehyd-Schwefelsäure als Nachweisreagenz) verfolgt. Nach Abschluß der Umsetzung wird das Pilzmycel abfiltriert, das resultierende Kulturfiltrat mit der gleichen Menge Dichlormethan extrahiert und eingedampft.

c) <u>Tranformationsprodukte</u> (Strukturen siehe Tabelle 1)

I) 1,9-Didesoxyforskolin (<u>1</u>) → 1 α-HO-DDF (<u>5</u>)
(DDF) (≙ 9-Desoxyforskolin)
→ 2 α-HO-DDF (<u>8</u>)
→ 3 α-HO-DDF (<u>9</u>)

II) 7-dAc-1,9-Didesoxforskolin (<u>11</u>)

7-dAc-9-Desoxyforskolin ( <u>12</u>)

III 1-Desoxyforskolin (3)→Forskolin ( <u>2</u>)

IV) Forskolin (<u>2</u>) → 3 α-HO-Forskolin (<u>10</u>)

→ 6Ac-7dAc-3α-HO-Forskolin (<u>13</u>)

Umsetzungsbeispiele mit N. crassa ATCC 10336

<u>Beispiel</u> <u>1</u> Biotransformation von 1,9-Didesoxyforskolin (DDF) Durchführung gemäß 1 b) 120 Stunden DC-Analyse der Reaktionsprudukte:

DDF 16,0 %

9-Desoxy-Forskolin 19,4 % ($ND_1$)

2α-HO-DDF 34,1 % ($ND_3$)

3α-HO-DDF 28,6 % ($ND_2$)

<u>Beispiel</u> <u>2</u> Biotransformation von 1-Desoxyforskolin Durchführung gemäß 1 b) 72 Stunden DC-Analyse der Reaktionsprodukte:

1-Desoxyforskolin 46 %

Forskolin 10%

N,$DF_2$ 39,9 %

<u>Beispiel</u> <u>3</u> Biotransformation von Forskolin Durchführung gemäß 1b) 72 Stunden DC-Analyse der Reaktionsprodukte:

Forskolin 79,2%

3α-HO-Forskolin 16,0 % ($NF_3$)

6Ac-7dAC-Forskolin 1,4 % ($NF_2$)

6Ac-7dAC-3α-HO-Forskolin 3,0 % ($NF_4$)

Beispiel 4

Eine nach Beispiel 1 durchgeführte Biotransformation von DDF ergab nach Extraktion mit Ethylacetat 97,8 mg Trockenprodukt. Diese Menge wurde in Dichlormethan gelöst, an 1 g Kieselgel 60 - (Grace, 31 µm, pH 7.0) imprägniert und dann in eine Vorsäule gefüllt. Über die Vorsäule wurde dann die Probe auf der Hauptsäule (102 ml Kieselgel, s.o.) bei einem Fluß von 2 ml/min mit Dichlormethan/Aceton = 15 / l chromatographiert. Nach ca. 22 h wurde die Polarität der mobilen Phase auf 10 / l erhöht. Die einzelnen Fraktionen (à 14 ml) wurden per DC ($CH_2Cl_2$ / Aceton = 7 / l; Detektion mit Anisaldehyd/$H_2SO_4$/Essigsäure wie weiter oben beschreiben) auf Substanzgehalt analysiert.

Neben unumgesetzten DDF wurden 2,1 mg NDI (Rf = 0.64), 4,2 mg ND2 (Rf = 0,46) und 7,9 mg eines Gemisches aus ND2 und ND3 (Rf = 0,36) erhalten. Dieses Gemisch wurde über präparative Dünnschichtchromatographie zu 3,5 mg ND2 und 4.0 mg ND3 aufgetrennt.

Beispiel 5

Eine nach Beispiel 3 durchgeführte Biotransformation von Forskolin ergab nach Extraktion mit Ethylacetat 57,1 mg Trockensubstanz.

Diese Menge wurde in 1,7 ml $CH_2Cl_2$ gelöst und über eine Kieselgelsäule (Merck, KG 60, 15 - 40 µm, 102,5 ml Säulenvolumen) mit der mobilen Phase $CH_2Cl_2$ / Aceton = 12 / l bei 2,5 ml/min chromatographiert. Nach 8 h wurde die mobile Phase mit dem Verhältnis 9 / l verwendet.

Nach DC[1]) erhält man im wesentlichen nicht umgesetztes Forskolin, 7-Desacetyl-forskolin und die neuen Substanzen NF2, NF3 und NF4.

NF2 3.0 mg Rf = 0,39

NF3 1,5 mg Rf = 0.23

NF4 -Rf = 0,11

Eine Nachreinigung der Zwischenfraktionen führte zu 0,9 mg NF3 und 0,7 mg NF4.

1) Keiselgelplatten, Merck, F254 10 x 20 cm mob. Phase: Petrolether / Ethylacetat = 2 / 1 Detektion: s. Beispiel 4

3α-Hydroxy-forskolin (NF3)

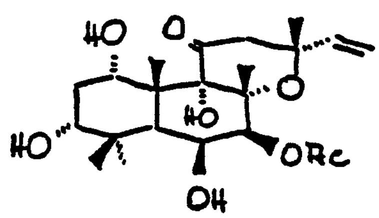

$^1$H-NMR (270 MH$_2$, $CDCl_3$, TMS) δ = 5.96 (dd, J = 10 Hz, J' = 18 Hz; 14-H), 5.57 (d, J = 4 Hz; 7 α-H), 5.30 (d, J = 18 Hz + Homoallylkopplung; 15 t-H), 4.99 (d, J = 10 Hz + Homoallylkopplung; 15 c-H), 4.65 (verbr. t, J = 4 Hz; 1 α-H), 4.41 (verbr. t, J = 5 Hz; 6 α-H), 3.52 (verbr. t. J = 4 Hz; 3 β-H), 2.85 (AB mit δA = 3.20 und $\delta_B$ = 2.50, $J_{AB}$ = 17 Hz; 12-H), 2.63 (d, J = 4 Hz; 5 α-H), 2.14 (AB mit $\delta_A$ = 2.34 und $\delta_B$ = 1.94, $J_{AB}$ = 16 Hz, zusätzlich J' = 3 Hz zu t; 2-H), 2.20 (s; OCO$\underline{CH_3}$), 1.88 -0.80 (restl. Signale, darin: $5_S$, je 3H bei δ = 1.73, 1.43, 1.37, 1.28 und 1.12, $\underline{CH}_3$).

MS m/z = 427 (18, $M^+$+H), 426 (4, $M^+$), 408 (78, $M^+$-$H_2O$), 391 (46), 380 (16), 331 (28), 125 - (100), 109 (90 %).

3α-Hydroxy-1,9-didesoxy-forskolin (ND2)

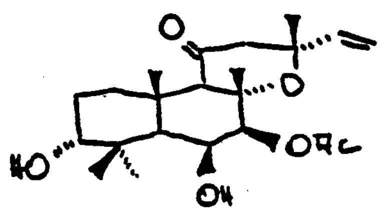

$^1$H-NMR (270 MHz, $CDCl_3$, TMS) $\delta$ = 5.95 (dd, 10 Hz, J' = 18 Hz; 14-H), 5.27 (d, J = 18 Hz + Homoallylkopplung; 15 t-H), 5.14 (d, J = 4 Hz + 7 $\alpha$-H), 5.06 (d, J = 10 Hz + Homoallylkopplung; 15 c-H), 4.21 (verbr. s; 6 $\alpha$-H), 3.38 (verbr. s, 3 $\beta$-H), 2.82 (s; 9 $\alpha$-H), 2.60 (AB mit $\delta A$ = 2.65 und $\delta_B$ = 2.55, $J_{AB}$ = 16 Hz; 12-H), 2.30 -2.00 (m, 5 H darin 2.21, s, $OCOC\underline{H}_3$), 1.78 -0.75 (rest. H, darin 4s: 1.52, 1.43, 1.22, 1.03 für $C\underline{H}_3$)

MS m/z = 379 ($M^+$-$CH_3$), 361 ($M^+$-$CH_3$-$H_2O$), 334 $M^+$-AcOH), 319 ($M^+$-$CH_3$-AcOH), 251, 222, 167, 151, 95, 71 %.

Die Figuren 1 und 2 zeigen die H-NMR-Signale der beiden Verbindungen ND2 bzw. NF3 im Bereich $\delta$ = 1.80 -6.50.

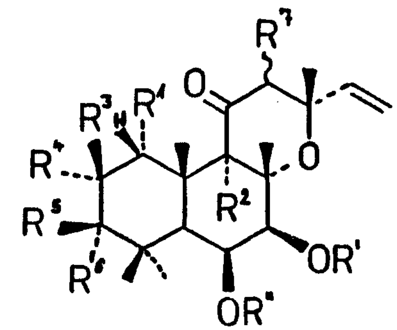

| | | R' | R'' | $R^1$ | $R^2$ | $R^3$ | $R^4$ | $R^5$ | $R^6$ | $R^7$ | |
|---|---|---|---|---|---|---|---|---|---|---|---|
| 1 | | Ac | H | H | H | H | H | H | H | H | 1,9-Didesoxy-forskolin |
| 2 | | Ac | H | OH | OH | H | H | H | H | H | Forskolin |
| 3 | | Ac | H | H | OH | H | H | H | H | H | 1-Desoxy-forskolin |
| 4 | ($M_1$) | Ac | H | H | H | H | H | — O — | | H | 3-Keto-DDF |
| 5 | ($M_{1.2}$, $ND_1$) | Ac | H | OH | H | H | H | H | H | H | 1 α -HO-DDF |
| 6 | ($M_2$) | Ac | H | H | H | OH | H | H | H | H | 2 β -HO-DDF |
| 7 | | Ac | H | H | H | H | H | OH | H | H | 3 β -HO-DDF |
| 8 | ($M_{3.1}$,$D_3$) | Ac | H | H | H | H | OH | H | H | H | 2 α -HO-DDF |
| 9 | (ND2) | Ac | H | H | H | H | H | H | OH | H | 3 α -HO-DDF |
| 10 | (NF3) | Ac | H | OH | OH | H | H | H | OH | H | 3 α -HO-Forskolin |
| 11 | | H | H | H | H | H | H | H | H | H | 7-dAc-DDF |
| 12 | | H | H | OH | H | H | H | H | H | H | 7-dAc-9-Desoxy-forskolin |
| 13 | (NF4) | H | Ac | OH | OH | H | H | H | OH | H | umgesch. 3α-HO-Forskolin |
| 14 | ($M_4$) | Ac | H | H | H | H | H | H | H | OH | 12-HO-DDF |

## Ansprüche

1. Verfahren zur mikrobiellen Hydroxylierung oder Oxidation von Labdan-Terpenoiden der Formel I

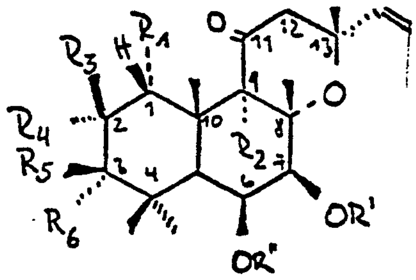

worin $R_1$ und $R_2$Wasserstoff oder Hydroxy, $R_3$ bis $R_6$ Wasserstoff, $R_2$ Wasserstoff oder Hydroxy, R' die Acetylgruppe oder Wasserstoff und R" Wasserstoff bedeuten, dadurch gekennzeichnet, daß man auf eine der genannten Verbindungen der Formel I als Substrat in einem üblichen Nährmedium einen Mikroorganismus-Stamm Neurospora crassa einwirken läßt und die gebildeten Verbindungen nach Beendigung der Fermentation aus der Kulturbrühe durch Extraktion mit einem organischen Lösungsmittel und anschließende Chromatographie und/oder Kristallisation isoliert.

2. Verfahren gemäß Anspruch 1, dadurch gekennzeichnet, daß man den Stamm Neurospora crassa ATCC 9279, ATCC 9344, ATCC 10336 oder 10780 verwendet.

3. Verfahren gemäß Anspruch 2, dadurch gekennzeichnet, daß man die Fermentation 3 -5 Tage bei 25° -30°C unter Schütteln durchführt und das Kulturfiltrat mit Dichlormethan extrahiert.

4. Verfahren gamäß Anspruch 3, dadurch gekennzeichnet, daß man bei 28°C fermentiert.

5. Verfahren gamäß den Ansprüchen 1 -4, dadurch gekennzeichnet, daß die Hydroxylierung in 1-, 2-und/oder 3-Stellung erfolgt.

6. 3α-Hydroxyforskolin.

7. Arzneimittel, enthaltend 3α-Hydroxyforskolin.

Patentansprüche für den Vertragsstaat: AT

1. Verfahren zur mikrobiellen Hydroxylierung oder Oxidation von Labdan-Terpenoiden der Formel I

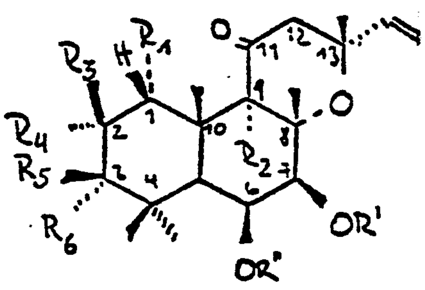

worin $R_1$ und $R_2$ Wasserstoff oder Hydroxy, $R_3$ bis $R_6$ Wasserstoff, $R_2$ Wasserstoff oder Hydroxy, R' die Acetylgruppe oder Wasserstoff und R" Wasserstoff bedeuten, dadurch gekennzeichnet, daß man auf eine der genannten Verbindungen der Formel I als Substrat in einem üblichen Nährmedium einen Mikroorganismus-Stamm Neurospora crassa einwirken läßt und die gebildeten Verbindungen nach Beendigung der Fermentation aus der Kulturbrühe durch Extraktion mit einem organischen Lösungsmittel und anschließende Chromatographie und/oder Kristallisation isoliert.

2. Verfahren gemäß Anspruch 1, dadurch gekennzeichnet, daß man den Stamm Neurospora crassa ATCC 9279, ATCC 9344, ATCC 10336 oder 10780 verwendet.

3. Verfahren gemäß Anspruch 2, dadurch gekennzeichnet, daß man die Fermentation 3 -5 Tage bei 25° -30°C unter Schütteln durchführt und das Kulturfiltrat mit Dichlormethan extrahiert.

4. Verfahren gemäß Anspruch 3, dadurch gekennzeichnet, daß man bei 28°C fermentiert.

5. Verfahren gemäß den Anspüchen 1 -4, dadurch gekennzeichnet, daß die Hydroxylierung in 1-, 2-und/oder 3-Stellung erfolgt.

FIG.1

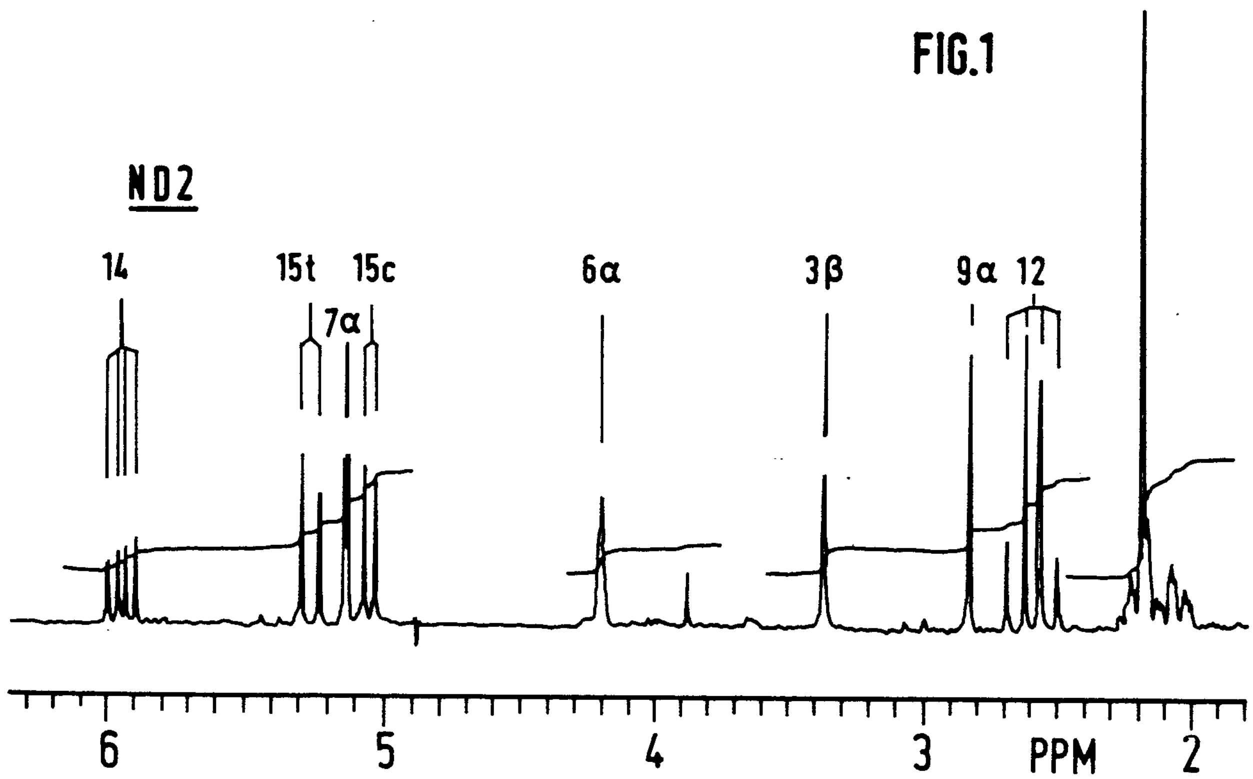
ND2
14
15t
7α
15c
6α
3β
9α
12
6
5
4
3
PPM
2

FIG.2

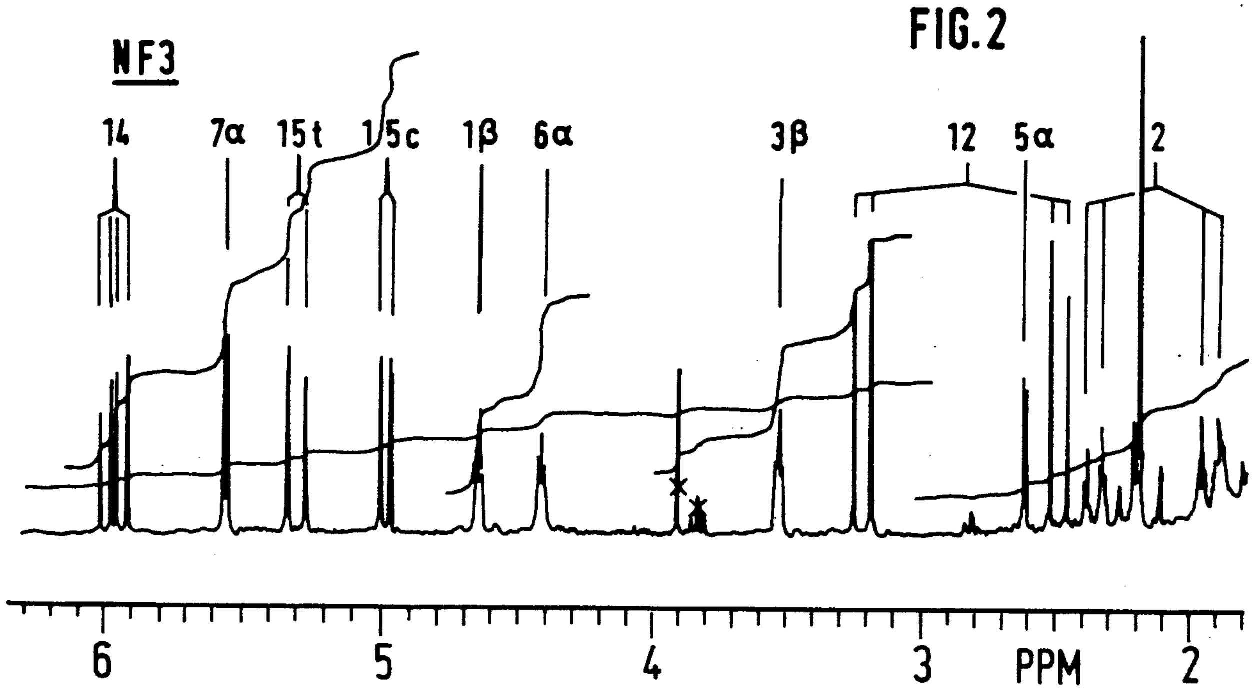
NF3
14
7α
15t
15c
1β
6α
3β
12
5α
2
6
5
4
3
PPM
2